# EUROPEAN PATENT APPLICATION

(11) **EP 1 004 317 A1**
(43) Date of publication of application: **31.05.2000**
(21) Application number: 98937785.8
(22) Date of filing: 12.08.1998
(51) Int. Cl.: A61K 45/00, A61K 31/17

(54) **REMEDIES FOR DISEASES ASSOCIATED WITH BONE RESORPTION**

(30) Priority: 12.08.1997 JP 25126497
(71) Applicant: CHUGAI SEIYAKU KABUSHIKI KAISHA, Tokyo, 115-8543 (JP)
(72) Inventor: ENDO, Koichi-Chugai Seiyaku Kabushiki Kaisha, Shizuoka 412-8513 (JP); KUSANO, Kenichiro-Chugai Seiyaku Kabushiki Kaisha, Shizuoka 412-8513 (JP)
(74) Representative: VOSSIUS & PARTNER
(86) International application number: JP9803581
(87) International publication number: WO9907412

(57) **Abstract**

Remedies for diseases associated with bone resorption wich contain as the active ingredient a selective iNOS inhibitor and are useful as remedies for osteoporosis, bone mass retaining agents, bone resorption inhibitors, tumor cell-bone metastasis inhibitors, remedies for nephritis, progression retardants for chronic renal failure, etc.

## Description

### TECHNICAL FIELD

This invention relates to drugs for treating bone resorption-associated diseases in the occurrence or development of which iNOS participates. Namely, the present invention relates to therapeutics of bone resorption-associated diseases containing selective iNOS inhibitors as the active ingredient. More particularly, it relates to therapeutics of bone resorption-associated diseases containing selective iNOS inhibitors as the active ingredient which are to be used as a therapeutic of osteoporosis, a bone mass-maintenance drug, a bone resorption retardant, an inhibitor of bone metastasis of tumor cells, a therapeutic of nephritis, a progression-retardant of chronic renal failure, etc.

### BACKGROUND ART

In recent years, it has been reported that nitric oxide (hereinafter referred to simply as NO), which has been considered as endothelium-derived relaxing factor, exerts various effects in a number of tissues (Nathan, C.F. & Hibbs, J.B.Jr., Curr. Opin. Immunol., 3:65-70, 1990, Liew, F.Y. & Cox, F.E.G., Immunol. Today, 12:A17-21, 1991). NO production is controlled by NO synthase (NOS) and it is known at present that NOS exists in three isoforms (Forstermann, U., Schmidt, H.H.H.W., et al., Biochem. Pharmacol. 42:1849-1857, 1991). It is pointed out that, among these isoforms, type II NOS (inducible NOS: iNOS) might participate in various diseases, since its expression is controlled by various cytokines (Moncada, S., et al., Pharmacol. Rev., 43:109-142, 1991, Nathan, C., FASEB J., 6:3051. 1992).

Recently, NO has become the center of attention as a bone metabolism regulator. It has been reported that nitroglycerin, which is an NO donor, counteracts the bone loss associated with ovariectomy (Wimalawansa S.J., et al., Bone 18:301-304, 1996). It has been also reported that pit formation serving as an indication of bone resorption is reduced by sodium nitropusside (SNP) which is another NO donor (Kasten T.P., et al., Proc. Natl. Acad. Sci. USA, 91:3569-3573, 1994). Based on these reports, it has been considered that NO would have therapeutic effect on osteoporosis (Schmidt, H.H.H.W. et al., J. Histochem. Cytochem. 40:1439-1456, 1992). On the other hand, it is known that inflammatory cytokines participating in osteoporosis (IL-1, TNF-α, etc.) enhance iNOS and thus accelerate NO production (Mika Hukkanen, et al., Endocrinology, 136;5445-5453, 1995).

Recently, Chow J.W.M., et al. reported in the American Society for Bone and Mineral Research that not iNOS but type I NOS (neural-constitutive NOS) and type III NOS (endothelial-constitutive NOS) are exclusively expressed in normal human bone tissues (Bone Miner. Res., 11, supplement 1:M354, 1996).

On the other hand, it is known that active bone resorption is observed in the attachment area of cancer cells upon bone tissues (Eilon G., Mundy GR., Nature, 276:726-728, 1978, Mundg GR. Raisz LG, et al., N. Engl. J. Med., 291:1041-1046, 1974).

Moreover, it has been known that the first signal of the induction of nephritis is the activation of NF-kB gene followed by the activation of iNOS gene (Xie, et al., J. Exp. Med., 177:1779-1784, 1994).

As described above, NO relates to various bone resorption-associated diseases typified by osteoporosis as well as bone metastasis of tumor cells, nephritis and chronic renal failure.

WO (International patent application opened public) 96-30350 discloses amidine derivatives which are useful as therapeutics of diseases in which NOS participates and osteoporosis is cited therein as an example of these diseases. However, nothing but an inhibitory activity on nNOS is disclosed in this patent as concrete specific data of these compounds.

As stated above, iNOS participates closely in bone metabolism and relates to bone resorption.

The therapeutics according to the present invention are efficacious entirely against bone resorption-associated diseases, in particular, osteoporosis, bone metastasis of tumor cells, nephritis, chronic renal failure, etc.

With the coming of the aging society, osteoporosis has attracted public attention not only as a medical problem but also as a serious social problem. Although it has been a practice to treat osteoporosis with the use of estrogen. calcitonin, active form of vitamin D, vitamin K, bisphosphonate, etc., these drugs are accompanied respectively by the problems of rejuvenation, drug resistance, hypercalcemia, hemolysis, drug resistance, etc. Thus, none of these drugs can establish a sufficient therapeutic effect from a clinical viewpoint.

On the basis of the relation between the attachment take of cancer cells upon bone tissues and bone resorption as described above, it is expected that the bone metastasis of tumor cells can be inhibited by controlling bone resorption. Accordingly, it can be said that bone metastasis of tumor cells also falls within the category of the bone resorption-associated diseases.

Since nephritis is induced by the activation of iNOS gene following the activation of NF-kB gene, it is highly meaningful in treating nephritis to selectively inhibit iNOS. Furthermore, the selective inhibition of iNOS is also meaningful in ameliorating uremic symptom in chronic renal failure and retarding the introduction of dialysis.

### DISCLOSURE OF THE INVENTION

The present invention, which relates to drugs for treating diseases in the occurrence or development of which iNOS participates, aims at providing therapeutics of bone resorption-associated diseases containing selective iNOS inhibitors as the active ingredient.

As the results of intensive studies on the assumption that selective inhibition of iNOS would contribute to the treatment of bone resorption-associated diseases, the present inventors have successfully found that selective iNOS inhibitors are useful in treating bone resorption-associated diseases, thus completing the present invention. Based on the above-mentioned finding on the relation of osteoporosis and NO and NOS, the present inventors assumed that iNOS would be expressed not in the ordinary state but in pathological states and, therefore, osteoporosis could be treated by selectively inhibiting iNOS. As the results of studies from this standpoint, they have found that selective iNOS inhibitors inhibit bone resorption observed in osteoporosis induced by IL-1, TNF-α, etc. and thus relieve decrease in bone mass.

Accordingly, the present invention provides therapeutics for bone resorption-associated diseases which contain as the active ingredient selective iNOS inhibitors.

The present invention also provides the above-described therapeutics of bone resorption-associated diseases which are to be used as a therapeutic of osteoporosis, a bone mass-maintenance drug, a bone resorption retardant, an inhibitor of bone metastasis of tumor cells, a therapeutic of nephritis, a progression retardant of chronic renal failure, etc.

### BRIEF DESCRIPTION OF DRAWING

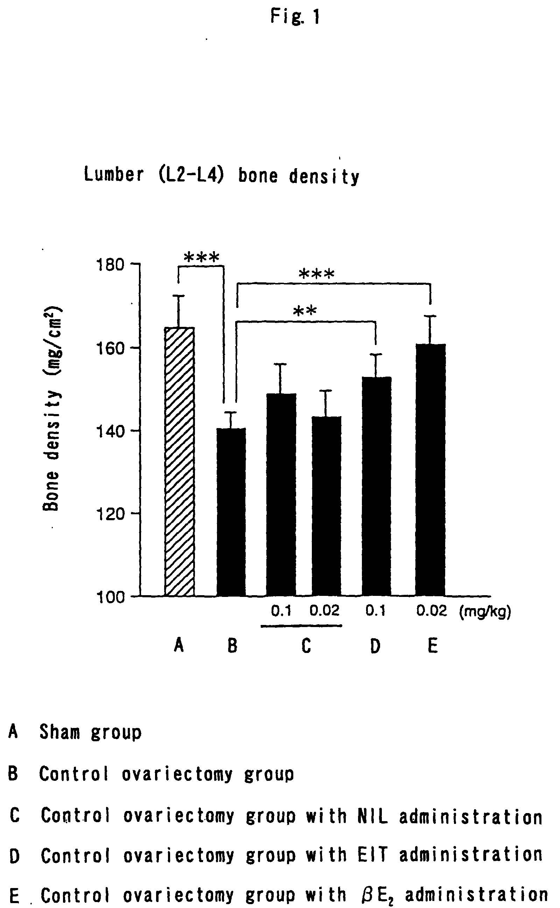

Fig. 1 is a graph which shows the inhibitory effect of the therapeutic according to the present invention on decrease in lumbar bone density.

### BEST MODE FOR CARRYING OUT THE INVENTION

The term "selective iNOS inhibitor" as used herein involves compounds showing extremely weak effect on two constitutive NOS isoforms (i.e., eNOS and nNOS), among the three NOS isoforms, but a selective inhibitory effect on the inducible one (i.e., iNOS). These compounds are not particularly restricted by difference in selectivity to eNOS and nNOS, so long as the inhibitory effect on iNOS exceeds those on eNOS and nNOS. More particularly speaking, it is preferable to use, for example, compounds satisfying any or all of the following requirements. When IC₅₀ levels on eNOS, nNOS or cNOS and iNOS are measured by the NOS-inhibitory activity determination method described in Proc. Natl. Acad. Sci. U.S.A. 88:365-369 (1991), the ratio eNOS/iNOS is 25 or above, nNOS/iNOS is 15 or above, or cNOS/iNOS is 15 or above. These compounds involve, for example, low-molecular weight synthetic compounds, peptide compounds and microbial products having the above-described effect. Examples thereof include isothiourea derivatives such as S-alkylisothiourea derivatives and cyclic isothiourea derivatives, amidine derivatives such as chain amidine derivatives and cyclic amidine derivatives, 2-aminopyridine derivatives and guanidine derivatives. Now, particular examples thereof will be cited.

Examples of S-alkylisothiourea derivatives are as follows:
S-ethylisothiourea (EIT) (Can. J. Physiol. Pharmacol., Vol. 73, p. 665, 1995);
S,S'-(1,3-phenylenebis(1,2-ethanedinyl))bis-isothiourea;
S,S'-(1,4-phenylenebis(1,2-ethanedinyl))bis-isothiourea;
S,S'-((2,5-dimethyl)-(1,4-phenylenebis(1,2-ethanedinyl))bis-isothiourea;
S-(3-methoxyphenethyl)isothiourea;
S-(3-(4-amidinothiomethyl)phenylmethyl)-propyl)isothiourea;
S,S'-(1,4-phenylenebis(1,3-propanedinyl))-bis-isothiourea (The Journal of Biological Chemistry, Vol. 269, No. 43, p. 26669, 1994); etc.

Examples of cyclic isothiourea derivatives are as follows:
3-amino-2-thia-4-aza-cis-bicyclo(4,4,0)-deca-3-ene hydrochloride;
2-amino-trans-5,6-dimethyl-5,6-dihydro-4H-1,3-thiazine hydrobromide;
3-amino-2-thia-4-aza-cis-bicyclo(4,4,0)-nona-3-ene methanesulfonate;
2-amino-trans-4,5-diemthyl-5,6-dihydro-4H-1,3-thiazine;
1(S)-6(R)-4-amino-3-thia-5-aza-cis-bycyclo(4,4,0)-deca-4-ene hydrochloride;
2-amino-cis-5,6-dimethyl-5,6-dihydro-4H-1,3-thiazine methanesulfonate;
S-((2-amino-thiazolyno)methyl)isothiourea;
2-amino-4-hydroxymethyl-thiazoline (WO96/14842);
2-amino-5,6-dihydro-6-methyl-4H-1,3-thiazine (AMT) (Can. J. Phusiol. Pharmacol., Vol. 73, p. 665, 1995); etc.

Examples of chain amidine derivatives are as follows:
L-N-6-(1-iminoethyl)lysine hydrochloride (NIL);
N-(5S-amino-6,7-dihydroxyheptyl)ethaneimidamide dihydrochloride;
N-(5S-amino-6,7-dihydroxy-6-methylheptyl)ethane-imidamide dihydrochloride dihydrate;
N-(5S-amino-6,7-dihydroxyoctyl)ethaneimidamide dihydrochloride hydrate;
3S-amino-7-((1-iminoetyl)amino)heptanoic acid (WO95/24382);
2-amino-6-(1-iminoethylamino)-4,4'-dioxo-4-thiahexanoic acid;
2-amino-6-(1-imino-2-fluoroethylamino)-4,4-dioxo-4-thiahexanoic acid dihydrobromide;
2-amino-6-(1-iminoethylamino)-4-oxo-4-thiahexanoic acid (WO95/34534); etc.

Examples of cyclic amidine derivatives are as follows:
7-[4,5-dihydro-3-phenylisoxazolyl-5-yl]methyl]-hexahydro-2H-azepin-2-imine monotrifluoroacetate;
(-)-hexahydro-7-(phenylmethyl)-2H-azepin-2-imine monohydrochloride;
(±)(trans)4-methyl-5-(phenylmethyl)pyrrolidin-2-imine monohydrochloride;
hexahydro-7-(phenylmethyl)-2H-azepin-2-imine monohydrochloride;
6-(cyclohexylmethyl)piperidin-2-imine monohydrochloride;
7-(cyclohexylmethyl)hexahydro-2H-azepin-2-imine monohydrochloride;
hexahydro-7-(3-phenylpropyl)-2H-azepin-2-imine monohydrochloride;
hexahydro-7-[(oxiran-2-yl)methyl]-2H-azepin-2-imine monohydrochloride;
hexahydro-7-(3-phenyl-2-propenyl)-2H-azepin-2-imine monohydrochloride (WO96/33175);
2-imino-5(S)-hydroxy-4(S)-methyl-piperidine hydrochloride;
4(S)-methyl-4a(S),8a(S)-decahydro-2-iminoquinoline hydrochloride;
4(R)-methyl-4a(R),8a(R)-decahydro-2-iminoquinoline hydrochloride;
4(S)-methyl-4a(S),7a(S)-perhydro-2-imino-1-pyridine hydrochloride;
4(R)-methyl-4a(R),7a(R)-perhydro-2-imino-1-pyridine hydrochloride;
5(R)-methyl-2-imino-piperidine hydrochloride;
4(R),5(R)-dimethyl-2-imino-piperidine hydrochloride;
2-imino-5(S)-methoxy-4(S)-methyl-piperidine hydrochloride;
4(R),5(S)-dimethyl-2-imino-piperidine hydrochloride;
trans-decahydro-2-iminoquinoline hydrochloride (WO96/14844); etc.

Examples of 2-aminopyridine derivatives are as follows:
2-amino-4,6-dimethyl-3-nitropyridine;
2-amino-6-benzylpyridine (WO96/02637);
2-amino-6-(2-aminoethyl)-4-methylpyridine (WO96/18616); etc.

The therapeutics of the present invention can be used in the form of various medicinal compositions prepared by blending the selective iNOS inhibitor, i.e., the active ingredient, with physiologically nontoxic solid or liquid pharmaceutical carriers. These medicinal compositions may be used in various dosage forms appropriate for administration methods. Examples of the dosage forms include tablets, granules, pills, capsules, solutions, syrups, suspensions, emulsions, ointments and patches. As the pharmaceutical carriers, use can be made of those commonly employed in the art, for example, fillers, binders, disintegrating agents, lubricating agents, coatings. solubilizing agents, emulsifiers, suspending agents, stabilizers and solvents. The therapeutics according to the present invention can be systemically administered as oral preparations or injections. Alternatively, they may be topically administered as external preparations, etc.

In the present invention, the dose of the selective iNOS inhibitor varies depending on the age and sex of the patient, the severity of the symptom, the administration route, etc. In general, it may be administered to an adult in a dose of 0.01 to 1,000 mg/day, preferably 0.1 to 100 mg/day.

The preventive effect of the inhibitor of bone metastasis of tumor cells according to the present invention can be confirmed by using a bone metastasis model prepared with the use of Hara cells originating in human pulmonary cancer which undergo bone metastasis at a high frequency.

The effects of the therapeutic of nephritis according to the present invention of retarding the development of nephritis and inhibiting the progression of chronic renal failure can be confirmed by using 5/6 nephrectomized rats.

### EXAMPLE

The present invention will be described in greater detail by reference to the following Example, but it should be understood that the invention is not construed as being limited thereto.

### EXAMPLE 1: Effect on ovariectomized rats

Female Wistar-Imamichi rats were subjected to ovariectomy (OVX). As test drugs, use was made of L-N-6-(1-iminoethyl)lysine hydrochloride (NIL) and S-ethylisothiourea (EIT) which are known as selective iNOS inhibitors. With respect to NOS inhibitory activity expressed in the ratio of IC₅₀, it is reported that NIL shows cNOS/iNOS of 28 while EIT shows nNOS/iNOS of 19.23 and eNOS/iNOS of 28.46 (Moore, W.M., et al., J. Med. Chem., 37:3886, 1994, Ross Tracey W., et al., Can. J. Pharmacol., 73:665-669, 1995). To confirm the validity of the test system, 17-β-estradiol (βE2) was also employed.

One day after OVX, the rats were divided into 7 groups each having 7 animals. The administration of the drug was started on the day 4 after OVX. As a control, a sham group (7 animals) was also employed. NIL was orally administered in a dose of 0.1 or 0.02 mg/kg (0.1 ml per 100 g of body weight) 5 times per week for 10 weeks.

Fig. 1 shows the results.

In this graph, the data are expressed in "mean ± standard deviation". According to Dunnet's multiple comparison method, the statistically significant differences from the control ovariectomy group are expressed as follows:
**: p < 0.01, ***: p < 0.001.

As Fig. 1 shows, the lumber bone density of the control ovariectomy group 24 hours after the final administration was significantly decreased to 85.2% (p < 0.001), when the lumber bone density of the sham group was referred to as 100%. In the EIT administration group, on the other hand, the decrease in the bone density was significantly inhibited, namely, 92.6% (p < 0.01).

In the 0.1 mg/kg NIL administration group, the decrease in the bone density was somewhat inhibited, i.e., 90.2%. In the 0.02 mg/kg NIL administration group, the bone density (86.8%) was almost comparable to that of the control OVX group.

Table 1 summarizes the biochemical test data of urine collected after the final administration.

**TABLE 1**

| | Biochemical parameter of urine | |
|---|---|---|
| | Dose (mg/kg) | D-pyr/Cre |
| Sham group | solvent | 7.66 ± 0.05** |
| Control OVX group | solvent | 11.83 ± 2.02 |
| OVX group with NIL administration | 0.1 | 10.43 ± 1.15 |
| | 0.02 | 8.70 ± 1.59** |
| OVX group with EIT administration | 0.1 | 9.95 ± 1.71 |
| OVX group with βE2 administration | 0.02 | 4.16 ± 1.45** |

According to Dunnet's multiple comparison method, the statistically significant differences from the control ovariectomy group are expressed as follows: **: p < 0.01, ***: p < 0.001.

Number of animals/group = 7. Mean ± standard deviation.

As Table 1 shows, the excretion of deoxypyridinoline (D-pyr) employed as a bone resorption marker was significantly accelerated (p < 0.01) in the control OVX group, compared with the sham group. In the 0.02 mg/kg NIL administration group, the acceleration was significantly inhibited (p < 0.01). In the EIT administration group, a tendency toward decrease was observed though no statistically significant difference was found.

### INDUSTRIAL APPLICABILITY

The present invention provides therapeutics of bone resorption-associated diseases containing selective iNOS inhibitors as the active ingredient.

## Claims

1. A therapeutic of bone resorption-associated diseases which contains a selective iNOS inhibitor as the active ingredient.

2. A therapeutic of bone resorption-associated diseases as claimed in Claim 1 which is to be used as a therapeutic of osteoporosis.

3. A therapeutic of bone resorption-associated diseases as claimed in Claim 1 which is to be used as a bone mass-maintenance drug.

4. A therapeutic of bone resorption-associated diseases as claimed in Claim 1 which is to be used as a bone resorption retardant.

5. A therapeutic of bone resorption-associated diseases as claimed in Claim 1 which is to be used as an inhibitor of bone metastasis of tumor cells.

6. A therapeutic of bone resorption-associated diseases as claimed in Claim 1 which is to be used as a therapeutic of nephritis.

7. A therapeutic of bone resorption-associated diseases as claimed in Claim 1 which is to be used as a progression retardant of chronic renal failure.
